# EUROPEAN PATENT APPLICATION

(11) **EP 2 133 418 A1**
(43) Date of publication of application: **16.12.2009**
(21) Application number: 07738479.0
(22) Date of filing: 07.03.2007
(51) Int. Cl.: C12N 5/06

(54) **CELL ISOLATION METHOD, CELL TESTING METHOD AND REAGENT KIT THEREFOR**

(71) Applicant: On-chip Cellomics Consortium, Tokyo 100-0006 (JP)
(72) Inventor: KIRA, Atsushi, Yokohama-shi Kanagawa 223-0065 (JP); HATTORI, Akihiro, Tokyo 114-0003 (JP); YASUDA, Kenji, Tokyo 135-0052 (JP)
(74) Representative: Prinz & Partner
(86) International application number: PCT/JP2007/055008
(87) International publication number: WO 2008/108006

(57) **Abstract**

The present invention provides a cell isolation method, a cell screening process, and a reagent kit therefor. In the present invention, the recognition substance is a polynucleotide specifically bound to a particular antigen that exists on the surface of a specific cell. This recognition substance is made to bind with a magnet of paramagnetic material. The specific cell is obtained by mixing a sample and the magnet, recovering the magnet through the use of magnetic force, applying nuclease, digesting the polynucleotide bound to the particular antigen of a specific cell, and using magnetic force to remove the magnet. According to the present invention, loss of cell functionality during the classification process can be avoided when cell identification or isolation is necessary.

## Description

### TECHNICAL FIELD

The present invention relates to a method for identification and isolation of cells. In particular, the present invention relates to cell identification/isolation for cell isolation used in regenerative medicine and cell implantation.

### BACKGROUND ART

Differentiation must be made in accordance with some sort of index when an attempt is made to identify or isolate cells. The following are examples of cell differentiation methods.

### 1) Classification by cell permeabilization dye:

Cell permeabilization dye can be used to classify each cell by the shape of the entire cell, the ratio of the nucleus to the entire cell, and the type and quantity of granules. When nuclei and intracellular granules are the targets of classification, they are often colored through the use of stain and detected. Examples of dyes used when the experimenter wants to see the nuclei are acetic orcein or acetocarmine, Papanicolaou stain, and DAPI stain. In addition to coloring using dye and detecting under visible light, fluorescence staining can also be used to observe fluorescent images.

Both a detection method wherein identification is made by visual observation under a microscope and a detection method wherein images are identified using a CCD camera or the like are in practical use. Examples include screening for bladder cancer or urethral cancer by screening for atypical cells that are expressed in urine, classification of atypical cells in the blood, and cancer screening through cytodiagnosis in tissue.

### 2) Classification by cell surface antigen (marker) stain:

Cell surface antigens, generally called "CD markers", are stained with fluorescence-labeled antibodies specific thereto and used in cell isolation by cell sorter, cancer screening by flow site meter and histological stain, etc. These cell surface antigens are, of course, heavily used not only in medical treatment, but also in cytophysiological research and in industrial cell usage.

These examples would be extremely useful if they were only for classifying cells in order to investigate the morphological differences thereof. In particular, method 2), which is based on surface antigen classification, can be used in minute classification, and has become indispensible in cell isolation by cell sorter. However, isolating cells and then attempting to culture and use the isolated cells presents a problem. Namely, modification by fluorescence-labeled antibodies of the surface antigens is irreversible, so cell function may be lost due to the fluorescence-labeled antibodies of the surface antigens that remain after isolation.

The labeled substance that is the isolation key is necessary to isolate cells. This labeled substance must identify a specific cell surface antigen and strongly bond thereto. This labeled substance must be bound to an identification substance comprising a microparticle and a phosphor for detecting that the labeled substance is bound to the cell.

Generally, an antibody for a surface antigen of the desired cell is used as the labeled substance that binds to the cell surface antigen. Monoclonal antibodies are often used as the antibodies. Polyclonal antibodies may, of course, also be used. F(ab), F(ab')₂, or F(ab') that removed the Fc section of the antibody are often used as antibodies that suppress nonspecific bonds or to prevent inadvertent activation of the complement system. An identification substance is covalently bound to these antibodies and F(ab), F(ab')₂, or F(ab'). Phosphors used as identification substances can be fluorescentally detected, and particles can also be optically detected. A method wherein a specific cell can be simultaneously detected and isolated by a magnet through the use of paramagnetic particles as identification particles is also in development.

However, the bond between an antibody, F(ab), F(ab')₂, or F(ab'), which is a labeling substance, and the surface antigen is strong as the binding constant being 10⁸M⁻¹ or larger. Accordingly, in equilibrium processing methods, it is extremely difficult to detach the labeled substance after cell isolation. Regardless, a method wherein the epitope of a surface antigen that is highly concentrated in terms of equilibrium is added as a free antigen is generally used as a method for dissolution of antigen/antibody bonds. The antibody, which is the labeling substance, can be disassociated from the cell by causing an interchange reaction between the free antigen and the cell surface antigen.

However, it is easy to imagine that exposure to a high concentration of surface antigens could greatly change or damage cell functionality. Alternatively, the antigen/antibody bond dissociates due to denaturation induced by a chaotropic ion such as 4M guanidine hydrochloride or hydrochloric acid acidification-induced acid denaturation. However, cells can not exist under such extreme conditions.

The present inventors proposed, as Japanese Patent Application No. 2004-226359, the use of a transporter that exists in specific cells as a new isolation method in order to avoid loss of cell function during the cell classification process. In other words, the labeling substance is integrated into the cell through the transporter on the cell surface, the labeling substance is caused to bond with a fluorescent dye as a labeling substance, and then cell classification is performed using the fluorescent dye that passed through the transporter and bound to the labeling substance. In this method, the integrated labeling substance can be reversibly drained from the transporter after isolation and collection, so post-isolation effect on the cell is minimal.

### DISCLOSURE OF THE INVENTION

Conventional cell isolation methods are: a method wherein a sheath flow cell is irradiated with a laser and the light that is scattered when the cell moves across the laser light is detected and a method wherein a cell is fluorescently labeled in advance and the fluorescent light that is produced when the laser moves across the cell is detected. With these methods, problems exist such as the generally high price of these flow cytometers and cell sorters, the large size of the devices, the fact that a large quantity of sample is necessary, the possibility for damage to the cell during production of droplets, and the fact that the sample cannot be directly observed.

There is also a need for a new cell identification method different from conventional cell isolation and identification by morphological cell classification or the fluorescence antibody method wherein irreversible cell identification is performed through staining with a cell surface antigen (marker). With this new cell identification method, a cell to which a labeling substance is bonded can be returned to the same state as that prior to the bonding of a labeling substance thereto after isolation. Alternatively, it is at least necessary that the effect on the cell be minimized by reversibly de-labeling a cell surface antigen that was once labeled with a labeling substance.

Accordingly, the present invention offers the following cell isolation method, cell isolation device, and cell isolation kit.
(1) A cell isolation method comprising:
   preparing a recognition element with a structure wherein a recognition material is bound to a carrier with paramagnetic properties, wherein said recognition material is a polynucleotide specifically bound to a specific antigen that exists on the surface of a specific cell;
   allowing the polynucleotide to bind to the specific antigen of the specific cell in the sample cell group in a solution in which the sample cell group and the recognition element are mixed,
   applying magnetism to a complex of the specific cell having the specific antigen and the recognition element;
   isolating the complex from the solution;
   degrading the polynucleotide bound to the specific antigen of the specific cell by a nuclease; and
   further removing the carrier with paramagnetic properties by means of magnetism so as to obtain the specific cell.
(2) A cell testing method comprising:
   bonding a polynucleotide capable of specifically binding to the surface antigen expressed in the cancer-derived cell to a surface antigen expressed in a cancer-derived cell in a sample cell group, wherein the polynucleotide is bonded through covalent bonding to a microparticle carrier that has paramagnetic property;
   isolating a complex of the cancer-derived cell and the recognition element through the use of a magnetic field;
   digesting the polynucleotide bound to the surface antigen expressed in the cancer-derived cell through the action of a nuclease on the complex;
   recovering the microparticle carrier having paramagnetic property through the use of a magnetic field to obtain the cancer-derived cell; and
   determining the presence of cancer by the presence of the cancer-derived cell.
(3) A cell isolation device comprising:
   a recognition element comprising a substance having paramagnetic capability bound to a polynucleotide that specifically bonds to a surface antigen expressed at a specific target cell;
   a mixing means configured to generate a conjugate of the specific cell and the polynucleotide from the mixture of the recognition element and a cell group sample;
   a magnetic field device for applying a magnetic field to the mixing means;
   a means for adding a nuclease in order to degrade the polynucleotide of the conjugate to the recognition element trapped in the magnetic field generated by the magnetic field device; and
   a means for recovering the specific cells isolated by the polynucleotide degradation.
(4) A reagent and testing kit comprising:
   the above-mentioned polynucleotide that specifically bonds to the surface antigen expressed at a specific cell being targeted;
   a recognition element which is the above-mentioned polynucleotide bound to a substance that has paramagnetic properties;
   a tube provided with a neodymium magnet array through which a sample suspension that is a mixture of a recognition element and a test specimen pass; and
   a nuclease for dissociating the complex comprising the above-mentioned recognition element and the above-mentioned specific cell.
(5) A method for isolating cells having a specific antigen on their surface comprising the following steps:
   mixing a modified polynucleotide wherein a carrier having paramagnetic properties is bound to a polynucleotide that specifically bonds to the above-mentioned antigen with a sample solution containing cells;
   applying magnetic force to the above-mentioned mixed sample solution to isolate cells to which the above-mentioned modified polynucleotide is bonded;
   adding a nuclease to the sample solution containing the above-mentioned isolated cells and degrading the above-mentioned polynucleotide;
   applying magnetic force to the above-mentioned sample solution after the above-mentioned polynucleotides have degraded; and
   removing the freed above-mentioned paramagnetic carrier.
(6) A method according to (5) wherein the polynucleotide is an RNA aptamer.
(7) A method according to (5) or (6) wherein the cell surface antigen is CD4.
(8) A method according to any one of (5) to (7) wherein the cell is a cancer-derived cell.
(9) The method according to (8) used for determining the existence of cancer.
(10) A cell isolation device used in the method according to any one of (5) to (9) above, said cell isolation device comprising:
   a means for retaining a sample solution containing a cell;
   a means for adding a modified polynucleotide to which a carrier having paramagnetic properties is attached, wherein said polynucleotide that specifically binds to the above-mentioned antigen in the above-mentioned sample solution;
   a means for applying a magnetic force to the above-mentioned sample solution;
   a means for adding a nuclease to the above-mentioned sample solution; and
   a means for recovering the above-mentioned isolated cells.
(11) A cell isolation kit used in the method according to any one of (5) to (9) above, said kit comprising:
   a polynucleotide that bonds to a specific antigen on the cell surface;
   a paramagnetic carrier for bonding with the above-mentioned polynucleotides
   a magnet;
   a nuclease for degradation of the above-mentioned nucleotide; and
   a retaining member for the retention of the sample solution.
(12) The kit according to (11) above wherein the above-mentioned polynucleotide and the above-mentioned paramagnetic carrier are bonded in advance.
(13) The kit according to (11) or (12) above wherein the above-mentioned magnet is positioned in advance at the above-mentioned retention member.
(14) The kit according to (13) above wherein the above-mentioned retention member is a tube having one or more neodymium magnets positioned longitudinally along its surface.

In the present invention, not only are substances that can be degraded under conditions that are mild to the identification substance of the surface antigen used, the identification substance of the surface antigen is degraded and then removed under physiological conditions so that the cells are not affected. Specifically, polynucleotides that can form various types of steric structure are used as the identification substance. This polynucleotide is generally called an aptamer. For example, many types of synthetic polynucleotides with a total length of 80 bases, wherein 20 bases on the 3' terminal side and the 5' terminal side are controlled known base sequences and the 40 bases in the central part are random sequences. These synthetic polynucleotides are passed through a column on whose interior is immobilized the surface antigen of the cells for which isolation is desired. As a result, polynucleotides of sequences having affinity to the surface antigen of the cells that desire isolation are trapped on the inner surface of the column. Polynucleotides that specifically bind to the cell surface antigen can be obtained by treating this column with alkali, isolating and collecting the trapped polynucleotides, and performing PCR amplification. Therefore, aptamers that are degradable identification substances for surface antigen can be obtained under mild conditions.

An evolutionary engineering method wherein fidelity is reduced on purpose so that the sequences change and affinity purification is repeated during PCR amplification can be used simultaneously in order to obtain a polynucleotide (aptamer) with greater specificity and stronger bond strength. There are also instances in which the base section that binds to the surface antigen is modified and charged, thereby increasing its bond strength. Alternatively, a nucleotide with a base whose sugar chain is modified may be used to increase bond strength.

The backbone structure of the obtained structure recognition-type polynucleotide may be either ribonucleotide-type or deoxyribonucleotide-type. Although ribonucleotide-type is generally advantageous because it can easily assume a wide variety of structures, when ribonucleotide-type is used there are also difficult cases in which there is inadvertent digestion due to RNase in the vicinity. This is because DNase does not exist in large quantities outside of cells and is easy to inactivate. Therefore, deoxyribonucleotide-type backbone structures are easier to work in.

The structure recognition-type polynucleotide (aptamer) obtained above is used as the recognition substance, paramagnetic particles are modified therewith, and recognition elements are thereby configured. If paramagnetic particles are too small in comparison with the size of the target cells, the paramagnetic particles will be incorporated into the cell when bonded, and will not only affect the condition of the cells, but will also make recovery by magnetic field difficult. However, as paramagnetic particle size increases, the probability of collision with cells in solution decreases, and the efficiency of cell recovery declines.

Therefore, the characteristics of the target cell and the collection purpose determine the size of the paramagnetic particle used as the recognition element carrier. If only the recovery amount of the target cell is of concern, a paramagnetic particle size at or below half the (estimated) average diameter of the target cell is used. Paramagnetic particles with a diameter of 4 µm, which is larger than a half of the average (estimated) diameter of the target cells, cannot be incorporated into a cell and the recovery rate is satisfactory. Paramagnetic particles with a diameter that exceeds 4 µm can be recovered quickly with a magnetic field and are therefore used when there are adequate target cells in a sample.

After a recognition element binds to a target cell, the target cell that is bound to the recognition element can be recovered through placement in a magnetic field.

Target cells bound to the recovered recognition elements are processed with a nuclease to digest and remove aptamer, which is a recognition substance that bonds to surface antigen. If recognition polynucleotides are ribonucleotide-type, they are digested with RNase. If recognition polynucleotides are deoxyribonucleotide-type, they are digested with DNase. Here, when using modified nucleotides to increase stability, it is extremely important to take care so that digestion with these nucleases is not completely obstructed. Nucleotide structures it is feared could obstruct nuclease effects should only be partially inserted into aptamer molecules. By performing the procedure in this way, for example, even if there is a section in which nuclease does not work, the aptamer, when viewed as a whole, will be adequately digested into low molecules. Subsequently, pure target cells can be isolated by using a magnetic field to recover paramagnetic particles isolated from target cells.

Accordingly, the cell isolation method wherein the recognition elements are a combination of aptamer as a recognition substance and paramagnetic particles makes possible extremely easy cell isolation in which cells are reversibly labeled, and samples are supplied in a magnetic field.

Structure recognition-type polynucleotide (aptamer), which is the labeling substance for cell surface antigens, can easily be removed with a nuclease. In addition, Since RNase and DNase cannot permeate the cell membrane, intracellular RNA and DNA do not suffer any damage. Furthermore, since it is difficult to conceive that RNA and DNA are exposed on the cell surface, it is not believed that the structure recognition-type polynucleotide (aptamer) bound to the cell surface antigens is affecting the cells themselves. Further, an isolation method in which paramagnetic particles used as a recognition element carrier are used makes an easy isolation method possible. Accordingly, this does not require heavy equipment, and the alteration of cells due to isolation processing can be prevented.

### BRIEF DESCRIPTION OF THE DRAWINGS

Fig. 1 is a diagram explaining the isolation system of the present invention and the flow thereof.
Figs 2(A) and 2(B) are figures that show a device example and a method in which cell surface antigen CD4 presenting cells are isolated and recovered with a magnetic field using recognition elements comprising paramagnetic particles modified with RNA aptamer that recognizes cell surface antigen CD4.

### BEST MODE FOR CARRYING OUT THE INVENTION

According to one embodiment of the present invention, a method in which cells having a particular antigen on their surface are isolated is provided. This method comprises the step of mixing a modified polynucleotide wherein a carrier having paramagnetic properties is bound to a polynucleotide that specifically binds to the above-mentioned antigen with a sample solution containing cells; the step of applying magnetic force to the above-mentioned mixed sample solution to isolate cells to which the above-mentioned modified polynucleotide is bound; the step of adding a nuclease to the sample solution containing the above-mentioned isolated cells and degrading the above-mentioned polynucleotide; and the step of applying magnetic force to the sample solution after the above-mentioned polynucleotides have degraded and removing the freed paramagnetic carrier.

In a preferable embodiment, the above-mentioned polynucleotide is an RNA aptamer. In a more preferable embodiment, the above-mentioned cell surface antigen is CD4.

In a specific embodiment of the present invention, the above-mentioned cells are cancer-derived cells. This specific embodiment is used in a test to determine the existence of cancer (in other words, a cancer diagnosis method).

In another embodiment of the present invention, a cell isolation device for use in this type of cell isolation method is provided. The cell isolation device of the present invention comprises the following means: a means for retaining a sample solution containing a cell; a means for adding a modified polynucleotide to which a carrier having paramagnetic properties is bound in the above-mentioned sample solution wherein the polynucleotides specifically bind to the above-mentioned antigen; a means for applying a magnetic force to the above-mentioned sample solution; a means for adding nuclease to the above-mentioned sample solution; and a means for recovering the above-mentioned isolated cells.

In yet another embodiment of the present invention, a cell isolation kit that can be used in the practice of the cell isolation method of the present invention is provided. The kit of the present invention typically contains a polynucleotide that binds to a specific antigen of the cell surface, a paramagnetic carrier for binding with the above-mentioned polynucleotide, a magnet, a nuclease for degradation of the above-mentioned polynucleotide; and a retaining member for the retention of the sample solution.

Throughout the present specification, "cell surface antigen" means a molecule expressed (bound) at the surface of a cell membrane of a cell including a lymph cell such as a T cell or B cell, or antigen presenting cells, etc. (for example, dendritic cells, macrophages and B cells) that present T cells to antigens expressed in the form of a peptide on a cell surface into which antigens have been taken up. These are classified by CD (cluster of differentiation). Examples of cell surface antigens related to the present invention include but are not limited to CD4, CD8, CD34, CD44 and the like.

Throughout the present specification, "polynucleotide that specifically binds to an antigen" means a nucleic acid ligand that specifically binds to a cell surface antigen, and typically includes an RNA aptamer or a DNA aptamer.

Throughout the present specification, "nuclease" means an enzyme that digests nucleic acid. Representative examples include ribonuclease, which digests RNA, and deoxyribonuclease, which digests DNA.

Throughout the present specification, "paramagnetic carrier" includes, for example, a ferromagnetic body broken into pieces smaller than the magnetic domain structure and the like, and means a carrier comprising a substance that produces no magnetization when there is no external magnetic field, and, when a magnetic field is applied, exhibits magnetism that magnetizes in the direction of said application. Examples of paramagnetic carriers used in the present invention include but are not limited to, for example, the above-mentioned ferromagnetic body broken into pieces smaller than the magnetic domain structure, dispersed so that they do not bind with each other within a bead and hardened as polymer beads which are paramagnetic particles (beads) having a tosyl group and epoxy group surface and sold by Dynal Biotech, etc.

Throughout the present specification, the term "modified polynucleotide" means a polynucleotide to which a paramagnetic carrier is bonded, and that binds specifically to a cell surface antigen. The ways in which paramagnetic carriers bond to a polynucleotide include but are not limited to the examples below.

Before explaining the Examples of the present invention, a preparation method for an aptamer for cell surface antigen CD4 and a method for modifying paramagnetic particles are explained as an example of an aptamer useful as a recognition substance of the present invention. An aptamer for cell surface antigen CD4 described in the article "Staining of cell surface human CD4 with 3'-F-pyrimidine-containing RNA aptamers for flow cytometry" published in Nucleic Acids Research 26,3915-3924 (1998) is used as an aptamer, which is a recognition substance. This aptamer is ribonucleotide-type, in other words, an RNA aptamer.

In the above-mentioned paper, GDP-β-S is introduced at the 5' terminal of an RNA aptamer through in vitro transcription so that the aptamer can be identified by fluorescent light. In other words, a thiophosphoric acid group is inserted at the 5' terminal of the aptamer at this point in time. Biotin into which an acetyl iodide group has been introduced is reacted with this thiophosphoric acid group to obtain 5' biotinylated RNA aptamer.

5' terminal biotinylated RNA aptamer is added to streptavidin-coated paramagnetic particles. Streptavidin-coated paramagnetic particles are already on the market. For example, 2.8 µm diameter paramagnetic particles that have the product name Dynabeads M-28 Streptavidin can be obtained from Dynal Biotech. 5' biotinylated RNA aptamer is modified by paramagnetic particles due to avidin-biotin interaction. In this instance, the modification quantity of the aptamer can be controlled by controlling the amount of 5' biotinylated RNA aptamer added and the salt strength of the mixture solution.

Next, a method example is explained in which recognition substances are modified by paramagnetic particles through chemical bonding. For an RNA aptamer, the method described in the above-mentioned paper article may be used. However, an RNA aptamer prepared through chemical synthesis is used here. Even in the below methods, the mixing ratio and the salt strength of the mixture solution are controlled when an RNA aptamer is modified by paramagnetic particles.
(i) Amide bond: An amino group is introduced during RNA aptamer chemical synthesis. Paramagnetic particles with a carboxyl group introduced on their surface are prepared, the carboxyl group is converted into the form of active ester with carbodiimide, and then reacted with the 5' aminated RNA aptamer.
(ii) Aldehyde bond: An RNA aptamer with an aldehyde group introduced at its 5' terminal is prepared. After paramagnetic particles for the carboxyl group surface are prepared and the carboxyl group is converted into the form of active ester with carbodiimide, paramagnetic particles that reacted with hydrazine and a 5' aldehyde group RNA aptamer are reacted.
(iii) Disulfide bond: A divalent reagent such as N-(8-Maleimidocapryloxy)sulfosuccinimide is reacted with a synthetic RNA aptamer having an amino group introduced at its 5' terminal such that a malemide group that reacts with the SH group is introduced at the 5' terminal of the RNA aptamer. A paramagnetic particle with an SH group introduced on its surface is prepared. The introduction of an SH group is performed by using 2-iminothiolane to modify an amino group of the paramagnetic particles that have NH2 on their surface. An RNA aptamer into which a maleimide group has been introduced is reacted through mixing with the paramagnetic particles that have the SH group on their surface at pH7.
(iv) Other: In addition, paramagnetic particles (beads) having a tosyl group and epoxy group surface and sold by Dynal Biotech can be used to modify RNA aptamer through covalent bonding. Furthermore, using paramagnetic particles of the isothiocyanate group surface to modify 5' aminated RNA aptamer is also a useful means.

The preceding explained a preparation method in which an RNA aptamer is modified by paramagnetic particles. Even in the case of a DNA aptamer modified by deoxyribonucleotide, since an SH group or an amino group can be introduced at the 5' terminal in the same way as the above-mentioned RNA aptamer when a DNA aptamer is synthesized by a synthesizer, a DNA aptamer can be prepared as a recognition element in the same way. In addition to the above-mentioned RNA synthesis methods, an RNA aptamer can be synthesized as a single-strand DNA having a T7 promoter at the 5' terminal and an RNA polymerase may be used to perform RNA transcription thereafter in accordance with a conventional method.

### EXAMPLES

### (EXAMPLE 1)

A device example in which an RNA aptamer is used as a substance for the recognition of cell surface antigen CD4 and in which paramagnetic particles with a particle diameter of 3 µm are used in the isolation and recovery of cells bound to the RNA aptamer is explained hereinbelow. In other words, recognition elements composed of the above-described RNA aptamer and paramagnetic particles are used, and a magnetic field is utilized, to isolate and recover cell surface antigen CD4 presenting cells.

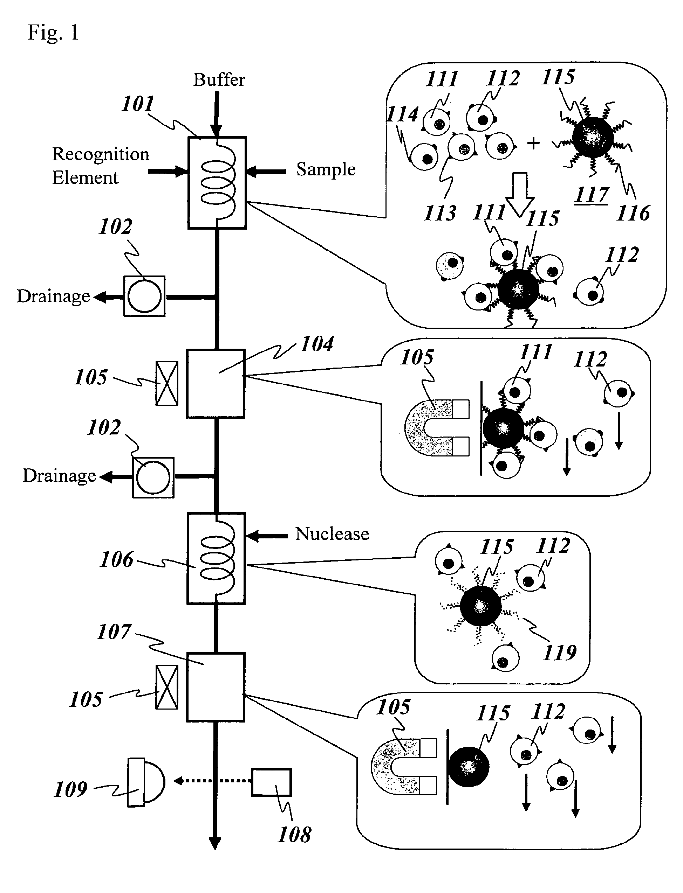

Fig. 1 is a diagram explaining the isolation system and flow thereof. 101 is a first mixing tank in which the sample cell groups which comprise the sample, a recognition element, and buffers for cleaning and dilution are introduced and mixed. In a first mixing tank 101, as indicated by the magnified image on the right side, a target cell 111 cell group which has a CD4 cell surface antigen 113 and is indicated by a ▲, a cell 112 cell group which has a cell surface antigen 114 other than CD4 and is indicated by •, a cell surface antigen CD4 recognition RNA aptamer 116, and recognition elements 117 modified by paramagnetic particles 115 are mixed. At this point, ingredients are mixed so that the concentration of recognition elements 117 is 110 nM. When adequate time passes, CD4 antigen 113 bonds with RNA aptamer 116 and target cells 111 and recognition elements 117 form an aggregate as a result. Unneeded drainage is controlled by valve 102 and flows out from a rear section of the first tank 101.

Next, a sample and a recognition element mixture solution are moved to the first magnetic field isolation tank 104. A magnet 105 is provided in first magnetic field isolation tank 104 in order to generate a magnetic field. Rare earth neodymium should be used for this magnet 105. Furthermore, this magnet is movable so that the strength of the magnetic field of first magnetic field isolation tank 104 can be adjusted. In the magnetic field, as indicated in the magnified image on the right side, target cells 111, which are bound to recognition element 117 having paramagnetic particles 115 as a carrier, are trapped in the magnetic field. Unbonded cells are controlled by valve 102 and run into the drain. As a result, only target cells 111 are recovered from the sample cell group.

Magnet 105 is released from the first magnetic field isolation tank 104, the magnetic field is now unapplied, and buffer is introduced from the upstream section. In consideration of the fact that processing will be performed with nuclease later, the buffer used here has a composition of 10 mM HEPES (pH 7.4), 0.15M NaCl, 2 mM MgCl₂, and 1 mg/ml BSA.

Next, the aggregate of target cells 111 and recognition elements 117 is mixed with 50 µ/ml of Benzonase (registered trademark) nuclease in second mixture tank 106. Since the RNA aptamer forms a steric structure, there are instances in which a type of nuclease such as ribonuclease A that only digests single-strand RNA does not provide adequate digestion. It is effective to use an enzyme that digests both single-strand and double-strand RNA. Here, the genetically-engineered, mass-produced, serratia marcescens-derived nuclease described in The Journal of Biological Chemistry 244, 5219-5225 (1969) is used. The product name is Benzonase (registered trademark) (EP Patent No. 0229866, U.S. Patent No. 5,173,418). This enzyme is easy to use on cells because it works at 37°C and its pH use range, 6 to 9, is in the neutral region. Highly concentrated phosphoric acid and univalent metallic ions cause a decline in enzymatic activity, so non phosphate buffer is used here. If phosphoric acid must be utilized, conditions are used wherein the potassium/sodium phosphate concentration is limited to 5 mM and 0.15 M NaCl, and 2 mM MgCl₂, 1 mg/ml BSA are included. Benzonaze (registered trademark) nuclease is used in a quantity of 10 to 100 u/ml. Although a mixture of ribonuclease A and ribonuclease T1 can also be used, Serratia marcescens-derived nuclease is more versatile.

As necessary, the use of plasma can be considered instead of buffer. However, if plasma is used, the nuclease inhibitor in plasma may have an effect. Consequently, it may be necessary to decrease the amount of Benzonase (registered trademark) nuclease additive in each plasma lot. Generally, when serum is used, good results are obtained with the use of 100 to 400 u/ml.

If nuclease is mixed with the combination of target cells 111 and recognition elements 117, the RNA aptamer will decompose as indicated by the magnified image on the right side (here, indicated by the broken line leading to reference mark 119) and target cells 111 will isolate from recognition elements 117.

After nuclease processing, the mixed sample, with target cells 111 and recognition elements 117 still isolated, is allowed to flow into second magnetic field isolation tank 107. As in first magnetic field isolation tank 104, a magnet 105 is also provided in second magnetic field isolation tank 107 in order to generate a magnetic field. As indicated in the magnified image on the right side, paramagnetic particles 115 are trapped by the magnetic field in the second magnetic field isolation tank 107, and then only the isolated target cells 111 flow away.

To enable counting of cells downstream from second magnetic field isolation tank 117, a light source 108 and a photon counter 109 are arranged on either side of a cell flow channel and the quantity of isolated target cells 111 is counted in real time. Scattered light detection may also be used as a method that can be used for measuring the quantity of cells.

Pure cell surface antigen CD4 presenting cells 111 can be collected by recovering the solution containing the recovered target cells, reciprocating the solution, and removing degradation product 119 of the recognition substance RNA aptamer. Centrifugation is used in the reciprocation of the cell supernatant. Centrifugation is performed at 3000 rpm for 15 minutes to precipitate the cells, and then the supernatant is disposed of.

Through the use of recognition elements comprising this sort of RNA aptamer-modified paramagnetic particles, the magnetic field makes extremely easy cell isolation possible. If RNA aptamers are bound to surface antigens, and the aptamers are removed by degradation with the ribonuclease that ordinarily exists in culture medium after cell recovery or excessively added ribonuclease, the aptamers will dissociate and the state of the proteins, etc. on the cell surface can be completely restored to its pre cell-labeled state without damage to the cell surface proteins, etc. that were used as aptamer bond targets.

As stated above, this technology exerts a revolutionary effect on the isolation and recovery of cells.

### (EXAMPLE 2)

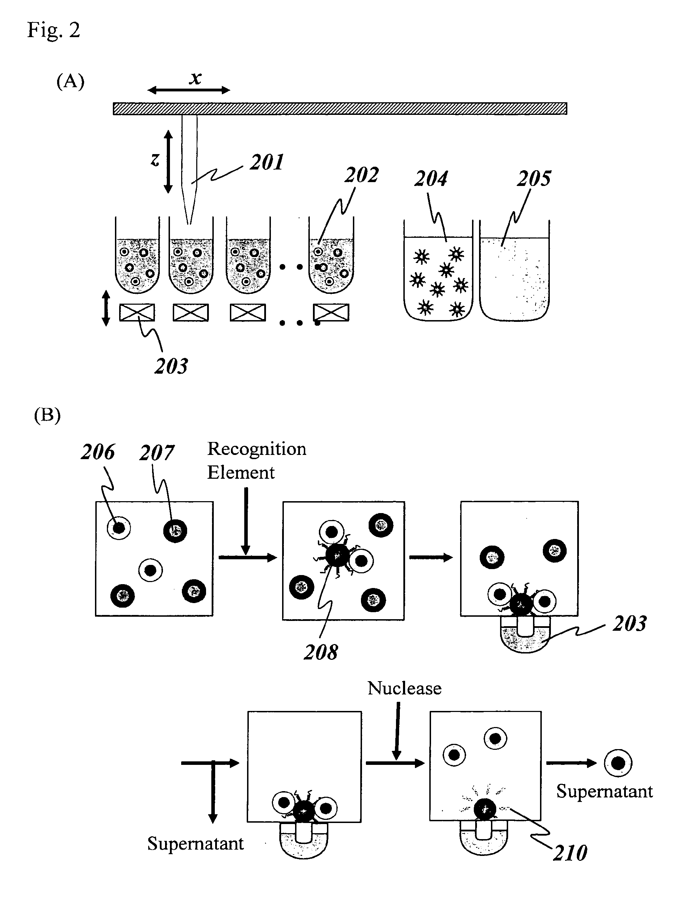

Figs 2(A) and 2(B) are figures that show a device example and a method in which cell surface antigen CD4 presenting cells are isolated and recovered with a magnetic field using recognition elements comprising paramagnetic particles modified with RNA aptamer that recognizes cell surface antigen CD4.

Fig. 2(A) is a conceptual diagram showing a screening device. Sample tube 202, dispensing mechanism 201, and magnetic field generation mechanism 203 is the smallest constituent unit. In addition, a tube retaining recognition element solution 204 (3 µm paramagnetic particles 208 modified by an RNA aptamer that recognizes cell surface antigen CD4 is intermixed), and a tube 205 that retains a nuclease solution are prepared. The movement of dispensing mechanism 201 is controlled in the x direction, y direction (perpendicular to sheet surface), and z direction. In addition, magnetic field generation mechanism 203 is movable, and the strength of the magnetic field within sample tube 202 can be controlled. Here, a rare earth magnet is used as a magnet for generating a magnetic field. Also, the magnetic field generation mechanism may be located to the side of the sample tube.

Fig. 2(B) shows a method in which this device is used to isolate and recover cell surface antigen CD4 presenting cells. Sample cell groups are inserted into sample tube 202. Cell surface antigen CD4 presenting cells 206 and cells 207, which have another surface antigen, are mixed together in sample cell groups. Dispensing mechanism 201 is used to dispense and stir recognition element solution 204. After a reaction is performed for an adequate amount of time while mixing by means of the dispensing mechanism, magnetic field generating mechanism 203 generates a magnetic field in sample tube 202. Cells that bound to recognition elements 208 are immobilized to the inner walls of the sample tube by magnetic force.

With a magnetic field applied, the supernatant is recovered with dispensing mechanism 201 and sample tube 202 is washed twice with a buffer or a culture solution. Subsequently, dispensing mechanism 201 adds nuclease solution 205 to sample tube 202. Adding nuclease digests the aptamer of recognition element 208 (indicated here by reference symbol 210). As a result, the cells 206 trapped on the walls of sample tube 202 and paramagnetic particles modified with aptamer and bound to aptamer are disassociated. By recovering the solution in which cells 206 were dissociated, cell surface antigen CD4 presenting cells are detached.

### (EXAMPLE 3)

A testing kit can be realized through the application of the present invention. Hereinbelow, a case is explained in which cell surface antigen CD44, which binds to an RNA aptamer, is the recognition substance and paramagnetic particles (particle diameter 1 µm) are used as the recognition element carrier. Explained here are separation detection of cells that express CD44 as a cell surface antigen and said cells are deprived, cancer-derived cells in fecal matter, and a testing kit therefor.

0.1 g of fecal matter, which is the test specimen, is suspended in a buffer of 1 mL of 10 mM PBS (pH 7.5), 0.15 M NaCL, 1% BSA and used as a sample. RNA aptamer-modified paramagnetic particles (recognition element) are added to the sample solution, which is gently stirred for 30 minutes. The suspension is fed into a tube with an internal diameter of 2mm, and magnetic particles moving inside the tube, which has a neodymium magnet array arranged at 1 cm intervals, are trapped. The collected paramagnetic particles are washed in culture fluid and Benzonase (registered trademark) nuclease is added.

After being gently stirred for 30 minutes, the mixture solution of the suspension and nuclease is fed into a neodymium magnet array, and the solution that passed through the array is collected and centrifuged to obtain cell surface aptamer CD44 revealing cells. Isolated live cells are cultured in a cell culture microchamber, for example, the cell culture microchamber proposed by the inventors of the present application and described in Japanese Patent Application No. 2004-305258. If the cells are cancerous, they will be able to withstand prolonged incubation, and dividing cells will soon start to appear.

In this way, cancer-derived cells are isolated from cells in fecal matter. Although cancerous sections cannot be determined by isolation of a cancer-derived cell from the fecal matter and incubation for a given period of time, the fact that there is a lesion in the body can be ascertained. Further, the effectiveness of anticancer drugs, etc. can be tested simultaneously.

Although the above explains illustrative examples of the present invention, various changes, modifications, and improvements could easily be thought of by one skilled in the art.

The above is merely illustrative of the principles of the present invention. One skilled in the art could arrive at various modifications without departing from the scope and spirit of the present invention.

### INDUSTRIAL APPLICABLITY

With the present invention, loss of cell function can be avoided during the classification process when it is necessary to identify or isolate cells. The present invention is especially useful as a cell identification/isolation method, a cell screening method and a reagent kit for cell isolation used in regenerative medicine and cell implants.

## Claims

1. A cell isolation method comprising:
preparing a recognition element with a structure wherein a recognition material is bound to a carrier with paramagnetic properties, wherein said recognition material is a polynucleotide specifically bound to a specific antigen that exists on the surface of a specific cell;
allowing the polynucleotide to bind to the specific antigen of the specific cell in the sample cell group in a solution in which the sample cell group and the recognition element are mixed,
applying magnetism to a complex of the specific cell having the specific antigen and the recognition element;
isolating the complex from the solution;
degrading the polynucleotide bound to the specific antigen of the specific cell by a nuclease; and
further removing the carrier with paramagnetic properties by means of magnetism so as to obtain the specific cell.

2. A cell testing method comprising:
bonding a polynucleotide capable of specifically binding to the surface antigen expressed in the cancer-derived cell to a surface antigen expressed in a cancer-derived cell in a sample cell group, wherein the polynucleotide is bonded through covalent bonding to a microparticle carrier that has paramagnetic property;
isolating a complex of the cancer-derived cell and the recognition element through the use of a magnetic field;
digesting the polynucleotide bound to the surface antigen expressed in the cancer-derived cell through the action of a nuclease on the complex;
recovering the microparticle carrier having paramagnetic property through the use of a magnetic field to obtain the cancer-derived cell; and
determining the presence of cancer by the presence of the cancer-derived cell.

3. A cell isolation device comprising:
a recognition element comprising a substance having paramagnetic capability bound to a polynucleotide that specifically bonds to a surface antigen expressed at a specific target cell;
a mixing means configured to generate a conjugate of the specific cell and the polynucleotide from the mixture of the recognition element and a cell group sample;
a magnetic field device for applying a magnetic field to the mixing means;
a means for adding a nuclease in order to degrade the polynucleotide of the conjugate to the recognition element trapped in the magnetic field generated by the magnetic field device; and
a means for recovering the specific cells isolated by the polynucleotide degradation.

4. A reagent and testing kit comprising:
the above-mentioned polynucleotide that specifically bonds to the surface antigen expressed at a specific cell being targeted;
a recognition element which is the above-mentioned polynucleotide bound to a substance that has paramagnetic properties;
a tube provided with a neodymium magnet array through which a sample suspension that is a mixture of a recognition element and a test specimen pass; and
a nuclease for dissociating the complex comprising the above-mentioned recognition element and the above-mentioned specific cell.

5. A method for isolating cells having a specific antigen on their surface comprising the following steps:
mixing a modified polynucleotide wherein a carrier having paramagnetic properties is bound to a polynucleotide that specifically bonds to the above-mentioned antigen with a sample solution containing cells;
applying magnetic force to the above-mentioned mixed sample solution to isolate cells to which the above-mentioned modified polynucleotide is bonded;
adding a nuclease to the sample solution containing the above-mentioned isolated cells and degrading the above-mentioned polynucleotide;
applying magnetic force to the above-mentioned sample solution after the above-mentioned polynucleotides have degraded; and
removing the freed above-mentioned paramagnetic carrier.

6. A method according to claim 5 wherein the polynucleotide is an RNA aptamer.

7. A method according to claim 5 or claim 6 wherein the cell surface antigen is CD4.

8. A method according to any one of claims 5 to 7 wherein the cell is a cancer-derived cell.

9. The method according to claim 8 used for determining the existence of cancer.

10. A cell isolation device used in the method according to any one of claims 5 to 9 above, said cell isolation device comprising:
a means for retaining a sample solution containing a cell;
a means for adding a modified polynucleotide to which a carrier having paramagnetic properties is attached, wherein said polynucleotide that specifically binds to the above-mentioned antigen in the above-mentioned sample solution;
a means for applying a magnetic force to the above-mentioned sample solution;
a means for adding a nuclease to the above-mentioned sample solution; and
a means for recovering the above-mentioned isolated cells.

11. A cell isolation kit used in the method according to any one of claims 5 to 9 above, said kit comprising:
a polynucleotide that bonds to a specific antigen on the cell surface;
a paramagnetic carrier for bonding with the above-mentioned polynucleotide;
a magnet;
a nuclease for degradation of the above-mentioned nucleotide; and
a retaining member for the retention of the sample solution.

12. The kit according to claim 11 wherein the above-mentioned polynucleotide and the above-mentioned paramagnetic carrier are bonded in advance.

13. The kit according to claim 11 or claim 12 wherein the above-mentioned magnet is positioned in advance at the above-mentioned retention member.

14. The kit according to claim 13 wherein the above-mentioned retention member is a tube having one or more neodymium magnets positioned longitudinally along its surface.
